(19) Europäisches Patentamt
European Patent Office
Office européen des brevets

(11) Publication number: **0 314 302 A2**

(12) # EUROPEAN PATENT APPLICATION

(21) Application number: 88308920.3

(22) Date of filing: 27.09.88

(51) Int. Cl.4: A01N 47/36 , C07D 213/80

(30) Priority: 28.09.87 US 101703
17.08.88 US 232465

(43) Date of publication of application:
03.05.89 Bulletin 89/18

(84) Designated Contracting States:
ES GR

(71) Applicant: E.I. DU PONT DE NEMOURS AND COMPANY
1007 Market Street
Wilmington Delaware 19898(US)

(72) Inventor: Liang, Paul Hsiao-Tseng
3428 Pebble Beach Drive
Wilmington Delaware 19808(US)
Inventor: Rardon, Patrick Lee
191 Ash Lane
Elkton Maryland 21921(US)
Inventor: Amuti, Kofi Sam
5412 Valley Green Drive
Wilmington Delaware 19808(US)

(74) Representative: Hildyard, Edward Martin et al
Frank B. Dehn & Co. European Patent Attorneys Imperial House 15-19 Kingsway
London WC2B 6UZ(GB)

(54) **Herbicidal o-carbomethoxy-sulfonylureas.**

(57) The compound 2-[[(4,6-dimethoxypyrimidin-2-yl)aminocarbonyl]-aminosulfonyl]-3-pyridinecarboxylic acid, methyl ester is useful as an agricultural chemical. It has high herbicidal activity, desirable spectrum of weed control and low residual activity. As such, it is particularly useful for fallow.

EP 0 314 302 A2

# HERBICIDAL o-CARBOMETHOXYSULFONYLUREAS

## Related Application

This application is a continuation-in-part of U.S. Serial No. 101,703, filed on September 28, 1987.

## Background of the Invention

A designated o-carbomethoxysulfonylurea is useful as an agricultural chemical. It has high herbicidal activity, a desirable spectrum of weed control and low residual activity, as such, it is particularly useful for fallow.

The compound of interest is disclosed in U.S. Patent 4,435,206, issued 3/6/84 and in EP-A-184,385, published 6/11/86, but not for all utilities.

The importance of cereal crops, such as wheat and barley, and plantation crops for feeding mankind is well known. Unfortunately, some of the known herbicides have high residual activity or are unsafe to crops. Thus, the crops cannot be easily rotated in an area in which known herbicides are present.

The present invention helps fill the need for herbicides with high herbicidal activity and low residual activity.

## Summary of the Invention

This invention pertains to the use of the compound of Formula I, its agriculturally suitable salts for broad spectrum weed control in fallow land and/or its use to control undesired vegetation in turf and plantation crops.

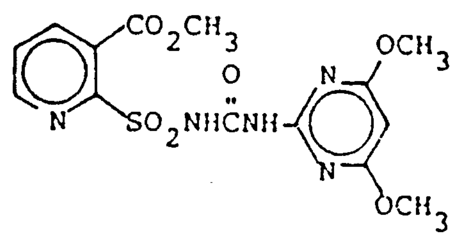

I

The compound is 2-[[(4,6-dimethoxypyrimidin-2-yl)aminocarbonyl]aminosulfonyl]-3-pyridinecarboxylic acid, methyl ester.

The compound also undergoes rapid soil dissipation and would ordinarily possess no recrop limitations.

## Detailed Description of the Invention

As part of the present invention, it has been found that unexpectedly high herbicidal activity with a desirable period of control for undesired vegetation in noncrop areas and fallow, as well as safety to turf and plantation crops, is exhibited by 2-[[(4,6-dimethoxypyrimidin-2-yl) aminocarbonyl]aminosulfonyl]-3-pyridinecarboxylic acid, methyl ester.

The following intermediates of Formula II are considered to be novel.

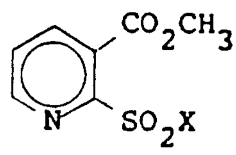

II

wherein

X is Cl, $NH_2$, $NHC(CH_3)_3$ and $HNC(O)OC_6H_5$.

Synthesis

The title compound of Formula I can be prepared by the reaction of 2-(aminosulfonyl)-3-pyridinecarboxylic acid, methyl ester and 4,6-dimethoxy-2-pyrimidinyl carbamic acid, phenyl ester in an inert aprotic solvent, such as toluene, acetonitrile, tetrahydrofuran, dichloromethane or dioxane, and in the presence of a base, such as 1,8-diazabicyclo-[5.4.0]undec-7-ene (DBU) or N,N-dimethylaniline.

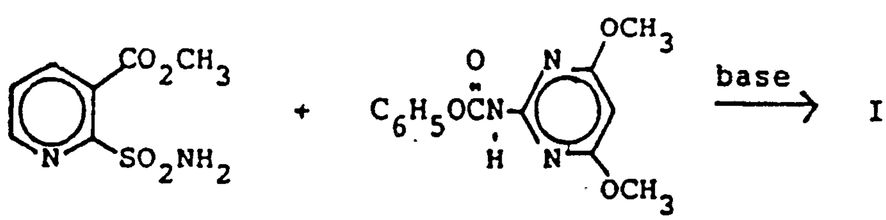


The following examples further illustrate the present invention.

Example 1

2-(Phenylmethylthio)-3-pyridinecarboxylic acid chloride

To a stirred suspension of 300 g of 2-(phenylmethylthio)-3-pyridinecarboxylic acid in benzene was added 268 mL of thionyl chloride, dropwise. The resultant yellow suspension was heated to reflux for 4 hours, allowed to cool to room temperature overnight and then concentrated under reduced pressure. The solid residue was washed with cold hexanes and dried in vacuo at room temperature to give 309.3 g of a tan powder, m.p. 94-96 °C; IR 1730 $cm^{-1}$ (C(O)); NMR (90 MHz, $CDCl_3$) $\delta$ 8.75 (dd, 2H, ArH), 7.5 (m, 6H, ArH), 4.5 (s, 2H, $CH_2$).

Example 2

2-(Phenylmethylthio)-3-pyridinecarboxylic acid, methyl ester

To a stirred solution of 4 L of methanol under a nitrogen atmosphere was added 450 g of 2-(phenylmethylthio)-3-pyridinecarboxylic acid chloride, followed by the dropwise addition of 382 mL of triethylamine. The resultant mixture was heated to reflux for two hours, then allowed to stir at room

temperature for 4 days. The reaction mixture was added to a battery jar containing 8 L of ice water. The resultant suspension was filtered through a sintered glass funnel and the residue was air dried and later dried in a vacuum oven at room temperature, to give 418.5 g of the desired product as a tan powder, m.p. 37-38 °C; IR 1715 $cm^{-1}$ (C(O)); NMR (90MHz, $CDCl_3$) δ 8.75 (dd, 1H, ArH), 8.3 (dd, 1H, ArH), 7.5 (m, 6H, ArH), 4.45 (s, 2H, $CH_2$) and 3.9 (s, 3H, $CH_3$).

## Example 3

2-[(1,1-Dimethylethyl)aminosulfonyl]-3-pyridinecarboxylic acid, methyl ester

A mixture of 100 g of 2-(phenylmethylthio)-3-pyridinecarboxylic acid, methyl ester, 500 mL of water and 500 mL of Freon® TF was cooled to 0 °C, and 70.9 mL of chlorine was then added via a gas dispersion tube. The resultant suspension was allowed to stir at 5 °C for 1 hour. The precipitate was collected and washed with cold Freon® TF, to give a white powder, m.p. 48-50 °C. This white solid was added to 1 L of dichloromethane at -50 °C under a nitrogen atmosphere. To this solution, cooled to -60 °C, was added dropwise, 226 g of t-butylamine. After warming to room temperature and dilution with 2 L of water, the organic layer was separated and the aqueous layer was extracted twice with 1 L of dichloromethane. The combined organic layers were washed with brine, dried over magnesium sulfate, filtered and the filtrate was concentrated under reduced pressure. The resultant yellow solid was slurried in cold n-butyl chloride, filtered and the solid collected to give 80.25 g of a white powder m.p. 160-161 °C; IR (nujol) 3160 $cm^{-1}$ (NH) and 1745 $cm^{-1}$ (C(O)); NMR (90.MHz, $CDCl_3$) δ 9.0 (dd, 1H, ArH), 8.3 (dd, 1H, ArH); 7.8 (m, 1H, ArH), 5.8 (bs, 1H, NH), 4.1 (s, 3H, $CH_3$) and 1.35 (s, 9H, $CH_3$).

## Example 4

2-(Aminosulfonyl)-3-pyridinecarboxylic acid, methyl ester

To 930 g of trifluoroacetic acid was added portionwise 50 g of 2-[(1,1-dimethylethyl)aminosulfonyl]-3-pyridinecarboxylic acid, methyl ester. The mixture was allowed to stir at room temperature for 3 days and then concentrated under reduced pressure. After addition of 150 mL of cold diethyl ether the resultant solid was collected. This solid was washed with cold ether and air dried to give 37.6 g of a white powder, m.p. 147-149 °C; IR (nujol) 3360 $cm^{-1}$ ($NH_2$), 1720 $cm^{-1}$ (C(O)) and 1070 $cm^{-1}$ ($SO_2$); NMR (DMSO-$d_6$) δ 9.0 (dd, 1H, ArH), 8.3 (dd, 1H, ArH), 7.9 (q, 1H, ArH), 7.2 (bs, 2H, $NH_2$) and 3.9 (s, 3H, $CH_3$).

## Example 5

2-[[(4,6-Dimethoxypyrimidin-2-yl)aminocarbonyl]aminosulfonyl]-3-pyridinecarboxylic acid, methyl ester

To a mixture of 346.4 g of 2-(aminosulfonyl)-3-pyridinecarboxylic acid, methyl ester and 485 g of 4,6-dimethoxy-2-pyrimidinyl carbamic acid, phenyl ester in 3,464 mL of dry acetonitrile was added dropwise 303 mL of DBU. After stirring the solution for 2 hours at room temperature, 3 teaspoons of activated carbon were added. The mixture was then filtered through Celite® to give a clear solution. The filtrate was diluted with 3.5 L of ice water and slowly concentrated hydrogen chloride was added until a pH of 3.5 was obtained. The solid was collected, washed with water, then diethyl ether and air dried to give 539.2 g of the desired product as a white powder, m.p. 158-161 °C; IR (nujol) 1735, 1745 $cm^{-1}$ (2 X C(O)), 1510 $cm^{-1}$ (NH) and 1080 $cm^{-1}$ ($SO_2$); NMR (90 MHz, $CDCl_3$) δ 11.8 (bs, 1H, NH), 8.9 (dd, 1H, ArH) 8.3 (dd, 1H, ArH), 7.75 (q, 1H, ArH), 7.6 (bs, 1H, NH), 5.9 (s, ArH, 1H) and 4.1 (s, 9H, $CH_3$).

Formulations

Useful formulations of the compound of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid diluent(s). More specifically, they will contain these ingredients in the following approximate proportions:

| | | Weight Percent* | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0° C. "McCutcheon's Detergents and Emulsifiers Annual," MC Publishing Corp., Ridgewood, New Jersey, as well as Sisely and Wood, "Encyclopedia of Surface Active Agents," Chemical Publishing Co., Inc., New York, 1964, list surfactants and recommended uses. All formulations can contain minor amounts of additives to reduce foaming, caking, corrosion, microbiological growth, and the like.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See Browning, "Agglomeration", Chemical Engineering, December 4, 1967, pp. 147ff. and "Perry's Chemical Engineer's Handbook" 5th Ed., McGraw-Hill, New York, 1973, pages 8 to 57 and following.

For further information regarding the art of formulation, see for example: U.S. Patent 3,235,361, column 6, line 16 through column 7, line 19 and Examples 10 through 41; U.S. Patent 3,309,192, column 5, line 43 through column 7, line 62 and Examples 8, 12, 15, 39, 41, 52, 53, 58, 132, 138 to 140, 162 to 164, 166, 167 and 169 to 182; U.S. Patent 2,891,855, column 3, line 66 through column 5, line 17 and Examples 1 to 4; Klingman, "Weed Control as a Science", John Wiley and Sons, Inc., New York, 1961, pages 81 to 96; and Fryer et al., "Weed Control Handbook", 5th Ed., Blackwell Scientific Publications, Oxford 1968, pages 101 to 103.

In the following Examples, all parts are by weight unless otherwise indicated.

## Example 6

### Wettable Powder

2-[[(4,6-dimethoxypyrimidin-2-yl)aminocarbonyl]aminosulfonyl]-3-pyridinecarboxylic acid, methyl ester 50%
sodium alkylnaphthalenesulfonate 2%
low viscosity methyl cellulose 2%
diatomaceous earth 46%

The ingredients are blended, coarsely hammer-milled and then air-milled to produce particles essentially all below 10 microns in diameter. The product is reblended before packaging.

## Example 7

### Granule

Wettable Powder of Example 6 5%
attapulgite granules 95%
(U.S.S. 20 to 40 mesh; 0.84 to 0.42 mm)

A slurry of wettable powder containing 25% solids is sprayed on the surface of attapulgite granules in a double-cone blender. The granules are dried and packaged.

Example 8

Extruded Pellet

2-[[(4,6-dimethoxypyrimidin-2-yl)aminocarbonyl]aminosulfonyl]-3-pyridinecarboxylic acid, methyl ester 25%
anhydrous sodium sulfate 10%
crude calcium ligninsulfonate 5%
sodium alkylnaphthalenesulfonate 1%
calcium/magnesium bentonite 59%

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 9

Low Strength Granule

2-[[(4,6-dimethoxypyrimidin-2-yl)aminocarbonyl]aminosulfonyl]-3-pyridinecarboxylic acid, methyl ester 1%
N,N-dimethylformamide 9%
attapulgite granules 90%
(U.S.S. 20 to 40 sieve, 0.42 to 0.84 mm)

The active ingredient is dissolved in the solvent and the solution is sprayed upon dedusted granules in a double cone blender. After spraying of the solution has been completed, the blender is allowed to run for a short period and then the granules are packaged.

Example 10

Aqueous Suspension

2-[[(4,6-dimethoxypyrimidin-2-yl)aminocarbonyl]aminosulfonyl]-3-pyridinecarboxylic acid, methyl ester 40%
polyacrylic acid thickener 0.3%
dodecylphenol polyethylene glycol ether 0.5%
disodium phosphate 1%
monosodium phosphate 0.5%
polyvinyl alcohol 1.0%
water 56.7%

The ingredients are blended and ground together in a sand mill to produce particles essentially all under 5 microns in size.

Example 11

Oil Suspension

2-[[(4,6-dimethoxypyrimidin-2-yl)aminocarbonyl]aminosulfonyl]-3-pyridinecarboxylic acid, methyl ester 35%
blend of polyalcohol carboxylic esters and oil soluble petroleum sulfonates 6%
xylene 59%

The ingredients are combined and ground together in a sand mill to produce particles essentially all below 5 microns. The product can be used directly, extended with oils, or emulsified in water.

## Example 12

Granule

2-[[(4,6-dimethoxypyrimidin-2-yl)aminocarbonyl]aminosulfonyl]-3-pyridinecarboxylic acid, methyl ester 80%
wetting agent 1%
crude ligninsulfonate salt (containing 5 to 20% of the natural sugars) 10%
attapulgite clay 9%

The ingredients are blended and milled to pass through a 100 mesh (0.149 mm opening) screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14 to 100 mesh (1410 to 149 microns), and packaged for use.

## Example 13

High Strength Concentrate

2-[[(4,6-dimethoxypyrimidin-2-yl)aminocarbonyl]aminosulfonyl]-3-pyridinecarboxylic acid, methyl ester 99%
silica aerogel 0.5%
synthetic amorphous silica 0.5%

The ingredients are blended and ground in a hammer-mill to produce a material essentially all passing a U.S.S. No. 50 screen (0.3 mm opening). The concentrate may be formulated further if necessary.

## Example 14

Wettable Powder

2-[[(4,6-dimethoxypyrimidin-2-yl)aminocarbonyl]aminosulfonyl]-3-pyridinecarboxylic acid, methyl ester 90%
dioctyl sodium sulfosuccinate 0.1%
synthetic fine silica 9.9%

The ingredients are blended and ground in a hammer-mill to produce particles essentially all below 100 microns. The material is sifted through a U.S.S. No. 50 screen and then packaged.

Example 15

Wettable Powder

2-[[(4,6-dimethoxypyrimidin-2-yl)aminocarbonyl]aminosulfonyl]-3-pyridinecarboxylic acid, methyl ester 20%
sodium ligninsulfonate 20%
montmorillonite clay 60%

The ingredients are thoroughly blended, coarsely hammer-milled and then air-milled to produce particles essentially all below 10 microns in size. The material is reblended and then packaged.

Example 16

Dust

2-[[(4,6-dimethoxypyrimidin-2-yl)aminocarbonyl]aminosulfonyl]-3-pyridinecarboxylic acid, methyl ester 10%
attapulgite 10%
Pyrophyllite 80%

The active ingredient is blended with attapulgite and then passed through a hammer-mill to produce particles substantially all below 200 microns. The ground concentrate is then blended with powdered pyrophyllite until homogeneous.

The compound of this invention may be used in combination with other commercial herbicides, plant growth regulants, insecticides or fungicides. Weed Research, Volume 26 (1986) pages 441 to 445, the disclosure of which is incorporated herein by reference, specifically names 294 commonly used herbicides and plant growth regulants. The following list exemplifies some of the herbicides suitable for use in mixtures.

| Common Name | Chemical Name |
|---|---|
| acetochlor | 2-chloro-N-(ethoxymethyl)-N-(2-ethyl-6-methylphenyl)acetamide |
| acifluorfen | 5-[2-chloro-4-(trifluoromethyl)phenoxy]-2-nitrobenzoic acid |
| acrolein | 2-propenal |
| alachlor | 2-chloro-N-(2,6-diethylphenyl)-N-(methoxymethyl)acetamide |
| ametryn | N-ethyl-N'-(1-methylethyl)-6-(methylthio)-1,3,5-triazine-2,4-diamine |
| amitrole | 1H-1,2,4-triazol-3-amine |
| AMS | ammonium sulfamate |
| asulam | methyl [(4-aminophenyl)sulfonyl]carbamate |
| atrazine | 6-chloro-N-ethyl-N'-(1-methylethyl)-1,3,5-triazine-2,4-diamine |
| barban | 4-chloro-2-butynyl 3-chlorocarbamate |
| benefin | N-butyl-N-ethyl-2,6-dinitro-4-(trifluoromethyl)benzenamine |
| bensulfuron methyl | 2-[[[[[(4,6-dimethoxy-2-pyrimidinyl)amino]-carbonyl]amino]sulfonyl]methyl]benzoic acid, methyl ester |
| bensulide | O,O-bis(1-methylethyl) S-[2-[(phenylsulfonyl)amino]ethyl] phosphorodithioate |
| bentazon | 3-(1-methylethyl)-(1H)-2,1,3-benzothiadiazin-4(3H)-one, 2,2-dioxide |
| benzofluor | N-[4-(ethylthio)-2-(trifluoromethyl)-phenyl]methanesulfonamide |
| benzoylprop | N-benzoyl-N-(3,4-dichlorophenyl)-DL-alanine |
| bifenox | methyl 5-(2,4-dichlorophenoxy)-2-nitrobenzoate |
| bromacil | 5-bromo-6-methyl-3-(1-methylpropyl)-2,4(1H,3H)pyrimidinedione |

| Common Name | Chemical Name |
|---|---|
| bromoxynil | 3,5-dibromo-4-hydroxybenzonitrile |
| butachlor | N-(butoxymethyl)-2-chloro-N-(2,6-diethylphenyl)acetamide |
| buthidazole | 3-[5-(1,1-dimethylethyl)-1,3,4-thiadiazol-2-yl]-4-hydroxy-1-methyl-2-imidazolidinone |
| butralin | 4-(1,1-dimethylethyl)-N-(1-methylpropyl)-2,6-dinitrobenzenamine |
| butylate | S-ethyl bis(2-methylpropyl)carbamothioate |
| cacodylic acid | dimethyl arsinic oxide |
| CDAA | 2-chloro-N,N-di-2-propenylacetamide |
| CDEC | 2-chloroallyl diethyldithiocarbamate |
| chloramben | 3-amino-2,5-dichlorobenzoic acid |
| chlorbromuron | 3-(4-bromo-3-chlorophenyl)-1-methoxy-1-methylurea |
| chlorimuron ethyl | 2-[[[[(4-chloro-6-methoxy-2-pyrimidinyl)amino]carbonyl]amino]sulfonyl]benzoic acid, ethyl ester |
| chloroxuron | N'-[4-(4-chlorophenoxy)phenyl]-N,N-dimethylurea |
| chlorpropham | 1-methylethyl 3-chlorophenylcarbamate |
| chlorsulfuron | 2-chloro-N-[[(4-methoxy-6-methyl-1,3,5-triazin-2-yl)amino]carbonyl]benzenesulfonamide |
| chlortoluron | N'-(3-chloro-4-methylphenyl)-N,N-dimethylurea |
| cinmethylin | exo-1-methyl-4-(1-methylethyl)-2-[(2-methylphenyl)methoxy]-7-oxabicyclo[2.2.1]heptane |
| clethodim | (E,E)-(±)-2-[1-[[(3-chloro-2-propenyl)oxy]imino]propyl]-5-[2-(ethylthio)propyl]-3-hydroxy-2-cyclohexen-1-one |

| Common Name | Chemical Name |
|---|---|
| clomazone | 2-[(2-chlorophenyl)methyl]-4,4-dimethyl-3-isoxazolidinone |
| cloproxydim | (E,E)-2-[1-[[(3-chloro-2-propenyl)oxy)-imino]butyl]-5-[2-(ethylthio)propyl]-3-hydroxy-2-cyclohexen-1-one |
| clopyralid | 3,6-dichloro-2-pyridinecarboxylic acid |
| CMA | calcium salt of MAA |
| cyanazine | 2-[[4-chloro-6-(ethylamino)-1,3,5-triazin-2-yl]amino]-2-methylpropanenitrile |
| cycloate | S-ethyl cyclohexylethylcarbamothioate |
| cycluron | 3-cyclooctyl-1,1-dimethylurea |
| cyperquat | 1-methyl-4-phenylpyridinium |
| cyprazine | 2-chloro-4-(cyclopropylamino)-6-(isopropylamino)-*s*-triazine |
| cyprazole | N-[5-(2-chloro-1,1-dimethylethyl)-1,3,4-thiadiazol-2-yl]cyclopropanecarboxamide |
| cypromid | 3',4'-dichlorocyclopropanecarboxanilide |
| dalapon | 2,2-dichloropropanoic acid |
| dazomet | tetrahydro-3,5-dimethyl-2H-1,3,5-thiadiazine-2-thione |
| DCPA | dimethyl 2,3,5,6-tetrachloro-1,4-benzenedicarboxylate |
| desmediphan | ethyl [3-[[(phenylamino)carbonyl]oxy]-phenyl]carbamate |
| desmetryn | 2-(isopropylamino)-4-(methylamino)-6-(methylthio)-*s*-triazine |
| diallate | S-(2,3-dichloro-2-propenyl)bis(1-methylethyl)carbamothioate |
| dicamba | 3,6-dichloro-2-methoxybenzoic acid |
| dichlobenil | 2,6-dichlorobenzonitrile |

| Common Name | Chemical Name |
|---|---|
| dichlorprop | (±)-2-(2,4-dichlorophenoxy)propanoic acid |
| dichlofop-methyl | (±)-2-[4-(2,4-dichlorophenoxy)phenoxy]-propanoic acid, methyl ester |
| diethatyl | N-(chloroacetyl)-N-(2,6-diethylphenyl)-glycine |
| difenzoquat | 1,2-dimethyl-3,5-diphenyl-1H-pyrazolium |
| dinitramine | $N^3$,$N^3$-diethyl-2,4-dinitro-6-(trifluoro-methyl)-1,3-benzenediamine |
| dinoseb | 2-(1-methylpropyl)-4,6-dinitrophenol |
| diphenamid | N,N-dimethyl-α-phenylbenzeneacetamide |
| dipropetryn | 6-(ethylthio)-N,N'-bis(1-methylethyl)-1,3,5-triazine-2,4-diamine |
| diquat | 6,7-dihydrodipyrido[1,2-a:2',1'-c]-pyrazinedium ion |
| diuron | N'-(3,4-dichlorophenyl)-N,N-dimethylurea |
| DNOC | 2-methyl-4,6-dinitrophenol |
| DPX-M6316 | 3-[[[[(4-methoxy-6-methyl-1,3,5-triazin-2-yl)amino]carbonyl]amino]sulfonyl]-2-thiophenecarboxylic acid, methyl ester |
| DSMA | disodium salt of MAA |
| endothall | 7-oxabicyclo[2.2.1]heptane-2,3-dicarboxylic acid |
| EPTC | S-ethyl dipropylcarbamothioate |
| ethalfluralin | N-ethyl-N-(2-methyl-2-propenyl)-2,6-dinitro-4-(trifluoromethyl)-benzenamine |

| Common Name | Chemical Name |
|---|---|
| ethofumesate | (±)-2-ethoxy-2,3-dihydro-3,3-dimethyl-5-benzofuranyl methanesulfonate |
| Express® | 2-[[[[N-(4-methoxy-6-methyl-1,3,5-triazine-2-yl)-N-methylamino]carbonyl]amino]-sulfonyl]benzoic acid, methyl ester |
| fenac | 2,3,6-trichlorobenzeneacetic acid |
| fenoxaprop | (±)-2-[4-[(6-chloro-2-benzoxazolyl)oxy]-phenoxy]propanoic acid |
| fenuron | N,N-dimethyl-N'-phenylurea |
| fenuron TCA | Salt of fenuron and TCA |
| flamprop | N-benzoyl-N-(3-chloro-4-fluorophenyl)-DL-alanine |
| fluazifop | (±)-2-[4-[[5-(trifluoromethyl)-2-pyridinyl]oxy]phenoxy]propanoic acid |
| fluazifop-P | (R)-2-[4-[[5-(trifluoromethyl)-2-pyridinyl]oxy]phenoxy]propanoic acid |
| fluchloralin | N-(2-chloroethyl)-2,6-dinitro-N-propyl-4-(trifluoromethyl)benzenamine |
| fluometuron | N,N-dimethyl-N'-[3-(trifluoromethyl)-phenyl]urea |
| fluorochlor-idone | 3-chloro-4-(chloromethyl)-1-[3-(trifluoromethyl)phenyl]-2-pyrrolidinone |
| fluorodifen | p-nitrophenyl α,α,α-trifluoro-2-nitro-p-tolyl ether |
| fluoroglycofen | carboxymethyl 5-[2-chloro-4-(trifluoromethyl)phenoxy]-2-nitrobenzoate |
| fluridone | 1-methyl-3-phenyl-5-[3-(trifluoromethyl)phenyl]-4(1H)-pyridinone |
| fomesafen | 5-[2-chloro-4-(trifluoromethyl)phenoxy]-N-(methylsulfonyl)-2-nitrobenzamide |

| Common Name | Chemical Name |
| --- | --- |
| fosamine | ethyl hydrogen (aminocarbonyl)-phosphate |
| glyphosate | N-(phosphonomethyl)glycine |
| haloxyfop | 2-[4-[[3-chloro-5-(trifluoromethyl)-2-pyridinyl]oxy]phenoxy]propanoic acid |
| hexaflurate | potassium hexafluoroarsenate |
| hexazinone | 3-cyclohexyl-6-(dimethylamino)-1-methyl-1,3,5-triazine-2,4(1H,3H)-dione |
| imazamethabenz | 6-(4-isopropyl-4-methyl-5-oxo-2-imidazolin-2-yl)-*m*-toluic acid, methyl ester and 6-(4-isopropyl-4-methyl-5-oxo-2-imidazolin-2-yl)-*p*-toluic acid, methyl ester |
| imazapyr | (±)-2-[4,5-dihydro-4-methyl-4-(1-methylethyl)-5-oxo-1H-imidazol-2-yl]-3-pyridinecarboxylic acid |
| imazaquin | 2-[4,5-dihydro-4-methyl-4-(1-methylethyl)-5-oxo-1H-imidazol-2-yl]-3-quinolinecarboxylic acid |
| imazethapyr | (±)-2-[4,5-dihydro-4-methyl-4-(1-methylethyl)-5-oxo-1H-imidazol-2-yl]-5-ethyl-3-pyridinecarboxylic acid |
| ioxynil | 4-hydroxy-3,5-diiodobenzonitrile |
| isopropalin | 4-(1-methylethyl)-2,6-dinitro-N,N-dipropylbenzenamine |
| isoproturon | N-(4-isopropylphenyl)-N',N'-dimethylurea |
| isouron | N'-[5-(1,1-dimethylethyl)-3-isoxazolyl]-N,N-dimethylurea |
| isoxaben | N-[3-(1-ethyl-1-methylpropyl)-5-isoxazolyl]-2,6-dimethoxybenzamide |
| karbutilate | 3-[[(dimethylamino)carbonyl]amino]-phenyl-(1,1-dimethylethyl)carbamate |

| Common Name | Chemical Name |
|---|---|
| lactofen | (±)-2-ethoxy-1-methyl-2-oxoethyl 5-[2-chloro-4-(trifluoromethyl)phenoxy]-2-nitrobenzoate |
| lenacil | 3-cyclohexyl-6,7-dihydro-1H-cyclopenta-pyrimidine-2,4(3H,5H)-dione |
| linuron | N'-(3,4-dichlorophenyl)-N-methoxy-N-methylurea |
| MAA | methylarsonic acid |
| MAMA | monoammonium salt of MAA |
| MCPA | (4-chloro-2-methylphenoxy)acetic acid |
| MCPB | 4-(4-chloro-2-methylphenoxy)butanoic acid |
| mecoprop | (±)-2-(4-chloro-2-methylphenoxy)-propanoic acid |
| mefluidide | N-[2,4-dimethyl-5-[[(trifluoromethyl)-sulfonyl]amino]phenyl]acetamide |
| methal-propalin | N-(2-methyl-2-propenyl)-2,6-dinitro-N-propyl-4-(trifluoromethyl)benzenamide |
| methabenz-thiazuron | 1,3-dimethyl-3-(2-benzothiazolyl)urea |
| metham | methylcarbamodithioic acid |
| methazole | 2-(3,4-dichlorophenyl)-4-methyl-1,2,4-oxadiazolidine-3,5-dione |
| methoxuron | N'-(3-chloro-4-methoxyphenyl)-N,N-dimethylurea |
| metolachlor | 2-chloro-N-(2-ethyl-6-methylphenyl)-N-(2-methoxy-1-methylethyl)acetamide |

| Common Name | Chemical Name |
|---|---|
| metribuzin | 4-amino-6-(1,1-dimethylethyl)-3-(methylthio)-1,2,4-triazin-5(4H)-one |
| metsulfuron methyl | 2-[[[[(4-methoxy-6-methyl-1,3,5-triazin-2-yl)amino]carbonyl]amino]sulfonyl]benzoic acid, methyl ester |
| MH | 1,2-dihydro-3,6-pyridazinedione |
| molinate | S-ethyl hexahydro-1H-azepine-1-carbothioate |
| monolinuron | 3-(p-chlorophenyl)-1-methoxy-1-methylurea |
| monuron | N'-(4-chlorophenyl)-N,N-dimethylurea |
| monuron TCA | Salt of monuron and TCA |
| MSMA | monosodium salt of MAA |
| napropamide | N,N-diethyl-2-(1-naphthalenyloxy)-propanamide |
| naptalam | 2-[(1-naphthalenylamino)carbonyl]-benzoic acid |
| neburon | 1-butyl-3-(3,4-dichlorophenyl)-1-methylurea |
| nitralin | 4-(methylsulfonyl)-2,6-dinitro-N,N-dipropylaniline |
| nitrofen | 2,4-dichloro-1-(4-nitrophenoxy)benzene |
| nitrofluorfen | 2-chloro-1-(4-nitrophenoxy)-4-(trifluoromethyl)benzene |
| norea | N,N-dimethyl-N'-(octahydro-4,7-methano-1H-inden-5-yl)urea 3aα,-4α,5α,7α,7aα-isomer |
| norflurazon | 4-chloro-5-(methylamino)-2-[3-(trifluoromethyl)phenyl]-3(2H)-pyridazinone |
| oryzalin | 4-(dipropylamino)-3,5-dinitrobenzenesulfonamide |

| Common Name | Chemical Name |
|---|---|
| oxadiazon | 3-[2,4-dichloro-5-(1-methylethoxy)-phenyl]-5-(1,1-dimethylethyl)-1,3,4-oxadiazol-2(3H)-one |
| oxyfluorfen | 2-chloro-1-(3-ethoxy-4-nitrophenoxy)-4-(trifluoromethyl)benzene |
| paraquat | 1,1'-dimethyl-4,4'-dipyridinium ion |
| pebulate | S-propyl butylethylcarbamothioate |
| pendimethalin | N-(1-ethylpropyl)-3,4-dimethyl-2,6-dinitrobenzenamine |
| perfluidone | 1,1,1-trifluoro-N-[2-methyl-4-(phenyl-sulfonyl)phenyl]methanesulfonamide |
| phenmedipham | 3-[(methoxycarbonyl)amino]phenyl (3-methylphenyl)carbamate |
| picloram | 4-amino-3,5,6-trichloro-2-pyridine-carboxylic acid |
| PPG-1013 | 5-[2-chloro-4-(trifluoromethyl)-phenoxy]-2-nitroacetophenone oxime-O-acetic acid, methyl ester |
| procyazine | 2-[[4-chloro-6-(cyclopropylamino)-1,3,5-triazine-2-yl]amino]-2-methylpropane-nitrile |
| profluralin | N-(cyclopropylmethyl)-2,6-dinitro-N-propyl-4-(trifluoromethyl)benzenamine |
| prometon | 6-methoxy-N,N'-bis(1-methylethyl)-1,3,5-triazine-2,4-diamine |
| prometryn | N,N'-bis(1-methylethyl)-6-(methylthio)-1,3,5-triazine-2,4-diamine |
| pronamide | 3,5-dichloro-N-(1,1-dimethyl-2-propyn-yl)benzamide |
| propachlor | 2-chloro-N-(1-methylethyl)-N-phenylacetamide |
| propanil | N-(3,4-dichlorophenyl)propanamide |
| propazine | 6-chloro-N,N'-bis(1-methylethyl)-1,3,5-triazine-2,4-diamine |

| Common Name | Chemical Name |
|---|---|
| propham | 1-methylethyl phenylcarbamate |
| prosulfalin | N-[[4-(dipropylamino)-3,5-dinitrophenyl]sulfonyl]-S,S-dimethylsulfilimine |
| prynachlor | 2-chloro-N-(1-methyl-2-propynyl)acetanilide |
| pyrazon | 5-amino-4-chloro-2-phenyl-3(2H)-pyridazinone |
| quizalofop ethyl | (±)-2-[4-[(6-chloro-2-quinoxalinyl)oxy]phenoxy]propanoic acid, ethyl ester |
| secbumeton | N-ethyl-6-methoxy-N'-(1-methylpropyl)-1,3,5-triazine-2,4-diamine |
| sethoxydim | 2-[1-(ethoxyimino)butyl]-5-[2-(ethylthio)propyl]-3-hydroxy-2-cyclohexen-1-one |
| siduron | N-(2-methylcyclohexyl)-N'-phenylurea |
| simazine | 6-chloro-N,N'-diethyl-1,3,5-triazine-2,4-diamine |
| sulfometuron methyl | 2-[[[[(4,6-dimethyl-2-pyrimidinyl)amino]carbonyl]amino]sulfonyl]benzoic acid, methyl ester |
| TCA | trichloroacetic acid |
| tebuthiuron | N-[5-(1,1-dimethylethyl)-1,3,4-thiadiazol-2-yl]-N,N'-dimethylurea |
| terbacil | 5-chloro-3-(1,1-dimethylethyl)-6-methyl-2,4(1H,3H)-pyrimidinedione |
| terbuchlor | N-(butoxymethyl)-2-chloro-N-[2-(1,1-dimethylethyl)-6-methylphenyl]acetamide |

| Common Name | Chemical Name |
|---|---|
| terbuthylazine | 2-(*tert*-butylamino)-4-chloro-6-(ethylamino)-*s*-triazine |
| terbutol | 2,6-di-*tert*-butyl-*p*-tolyl methylcarbamate |
| terbutryn | N-(1,1-dimethylethyl)-N'-ethyl-6-(methylthio)-1,3,5-triazine-2,4-diamine |
| thiobencarb | S-[(4-chlorophenyl)methyl] diethylcarbamothioate |
| triallate | S-(2,3,3-trichloro-2-propenyl) bis(1-methylethyl)carbamothioate |
| triclopyr | [(3,5,6-trichloro-2-pyridinyl)-oxy]acetic acid |
| tridiphane | 2-(3,5-dichlorophenyl)-2-(2,2,2-trichloroethyl)oxirane |
| trifluralin | 2,6-dinitro-N,N-dipropyl-4-(trifluoromethyl)benzenamine |
| trimeturon | 1-(*p*-chlorophenyl)-2,3,3-trimethylpseudourea |
| 2,4-D | (2,4-dichlorophenoxy)acetic acid |
| 2,4-DB | 4-(2,4-dichlorophenoxy)butanoic acid |
| vernolate | S-propyl dipropylcarbamothioate |
| xylachlor | 2-chloro-N-(2,3-dimethylphenyl)-N-(1-methylethyl)acetamide |

The herbicidal properties of the subject compounds were discovered in a number of greenhouse tests. The test procedures and results follow.

## Utility

The compound of the present invention is an active herbicide for selective and/or general broadleaf and grass weed control in non-crop situations, fallow, plantation crops such as coffee, tea, cocoa, oil palm, rubber, sugarcane, grapes, fruit trees, nut trees, banana, plantain, pineapple, citrus, cork, and certain conifers, and pasture and turf grasses such as Kentucky bluegrass, fescue, annual and perennial ryegrass, bermudagrass, bentgrass, and bahiagrass.

Compound I can be applied as a preemergence or postemergence treatment using techniques of banding, directed sprays or broadcast applications. By selecting the appropriate rate, the compound of this invention can be used in areas where complete control of all vegetation is desired, such as around fuel storage tanks, industrial storage areas, oil well sites, drive-in theatres, around billboards, highways, utilities, and railroad structures. Further, the compound's rapid soil dissipation allows it to be used in fallow fields where the ability to replant sensitive crops is desired. Alternatively, by selecting the proper rates and

adjuvants, the compound of this invention can be used for selective control of weeds in plantation crops such as coffee, tea, cocoa, oil palm, rubber, grapes, fruit trees, nut trees, banana, plantain, pineapple, citrus, cork, and sugarcane, certain conifers, and in turf and pasture grasses. In general, the compound of this invention is used at 0.5 to 500 g/ha with a preferred rate range of 1 to 250 g/ha. One skilled in the art can select the proper rate for a given situation.

The compound of this invention may be used in combination with other herbicides. It is particularly useful in combination with herbicides used in fallow, total vegetation control, plantation crops, certain conifers, pastures, and turfs. These herbicides include triazine, triazole, uracil, urea, amide, carbamate, bipyridylium, phenoxy, sulfonyl-urea, and imidazolinone types of chemistries. It may also be used in combination with mefluidide or glyphosate.

More preferred mixtures for use on fallow land would include DPX-M6316, Express®, difenzoquat, diclofop-methyl, paraquat, glyphosate and imazamethabenz.

The herbicidal properties of the subject compound were discovered in a number of greenhouse tests conducted as described below. Test results clearly demonstrate the various herbicidal uses of Compound I.

Test 1

Seeds of Bromus tectorum, Kochia scoparia, Salsoa kali, Avena fatua, Convolvulus arvensis, Secale cereale, Setaria viridis and Triticum aestivum were placed in 26-cm plastic pans containing a pasteurized sandy loam soil (pH 6.5, 1% organic matter). Plantings were maintained in the greenhouse for 18-28 days at which time the postemergence treatments were applied using a nonphytotoxic solvent as the carrier for the herbicide. The preemergence segment of the test was seeded immediately before herbicide application using Bromus tectorum, Kochia scoparia, Polygonum convolvulus, Salsoa kali, Avena fatua, Convolvulus arvensis, Secale cereale, Setaria viridis, Aegilops cylindrica, Amaranthus retroflexus, Chenopodium album, Hordeum vulgare, Triticum aestivum, Sorghum bicolor, and Zea mays. All treatments were maintained in the greenhouse for an additional 21 days at which time visual assessment of weed control was determined using a scale of 0 to 100 for each species where 0 represented no control and 100 represented complete control. Test results are shown in Table 1.

Table 1

| | Rate of Active Ingredient (g/ha) | | | | | |
|---|---|---|---|---|---|---|
| Species | 125 | 64 | 32 | 16 | 8 | 4 |
| POSTEMERGENCE | | | | | | |
| Kochia scoparia | 100 | 100 | 100 | 100 | 100 | 100 |
| Salsoa kali | 100 | 100 | 100 | 100 | 100 | 100 |
| Convolvulus arvensis | 100 | 100 | 100 | 100 | 100 | 90 |
| Avena fatua | 100 | 100 | 100 | 100 | 80 | 80 |
| Bromus tectorum | 100 | 100 | 100 | 100 | 100 | 100 |
| Secale cereale | 100 | 100 | 100 | 100 | 100 | 70 |
| Setaria viridis | 100 | 100 | 100 | 100 | 90 | 90 |
| Triticum aestivum | 100 | 100 | 100 | 100 | 100 | 90 |
| PREEMERGENCE | | | | | | |
| Amaranthus retroflexus | 100 | 100 | 100 | 90 | 90 | 70 |
| Chenopodium album | 100 | 100 | 100 | 90 | 90 | 60 |
| Convolvulus arvensis | 100 | 100 | 90 | 90 | 40 | 20 |
| Kochia scoparia | 100 | 100 | 100 | 100 | 80 | 80 |
| Polygonum convolvulus | 100 | 80 | 80 | 80 | 20 | 20 |
| Salsoa kali | 100 | 100 | 80 | 70 | 70 | 50 |
| Aegilops cylindrica | 100 | 100 | 80 | 60 | 50 | 30 |
| Avena fatua | 100 | 90 | 60 | 50 | 40 | 0 |
| Bromus tectorum | 100 | 90 | 90 | 80 | 70 | 50 |
| Hordeum vulgare | 90 | 80 | 80 | 70 | 60 | 50 |
| Secale cereale | 90 | 80 | 70 | 40 | 30 | 20 |
| Setaria viridis | 100 | 90 | 70 | 50 | 30 | 20 |
| Sorghum bicolor | 100 | 100 | 100 | 100 | 100 | 90 |
| Triticum aestivum | 100 | 100 | 100 | 100 | 100 | 70 |
| Zea mays | 100 | 90 | 90 | 90 | 50 | 40 |

Test 2

Rhizomes of Agropyron repens and Cirsium arvense were planted in 17.5-cm plastic pots and grown in the greenhouse for 5 months prior to herbicide application. Plants were 24 to 35 cm tall and releasing lateral buds at the time of treatment. Compound I was diluted in water containing 0.5% v/v non-ionic surfactant and applied to the plants. The degree of weed control was visually rated 36 days after application of the herbicide. Control ratings were based upon comparison to an untreated control using a scale of 0 to 100 where 0 indicated no effect and 10 represented death of all foliar portions with no regrowth apparent. Results are given in Table 2.

Table 2

| Treatment | Rate g/ha | Agropyron repens | Cirsium arvense |
|---|---|---|---|
| Compound I | 64 | 95 | 50 |
| | 32 | 90 | 50 |
| | 16 | 85 | 40 |
| Control ratings 36 DAT | | | |

Test 3

Seeds of crabgrass (Digitaria spp), guinea grass (Panicum maximum) and narrowleaf panicum (P. maximum) were planted in 15-cm plastic pots. The weeds were treated postemergence with compound I, formulated in a non-phytotoxic solvent, when the plants had 3 or more leaves and had tillered. The degree of weed control was visually rated 17 days after compound application (DAT). Control ratings were based on a scale of 0 to 100 where 0 = no effect, 20 = minimal control and 100 = complete control. Results are given in Table 3.

Table 3

| | | Control Rating 17 DAT | | |
|---|---|---|---|---|
| Treatment | Rate g/ha | Crabgrass | Guinea Grass | Narrowleaf panicum |
| Compound I | 32 | 100 | 70 | 90 |
| | 16 | 70 | - | - |
| | 8 | 50 | - | - |
| Check | 0 | 0 | 0 | 0 |
| - = not determined. | | | | |

Test 4

The object of this test was to evaluate the efficacy of the compound of this invention on weeds that infest some plantation crops at different growth stages of the weeds. Asystasia intrusa, Mikania cordata, goldenrod Solidago spp, Boston fern Nephrolepis exalata, and lalang Imparata cylindrica were planted in 15-cm plastic pots. Plants were sprayed postemergence with Compound I in a non-phytotoxic solvent. The Asystasia plants ranged in growth from 4 leaves to 9 leaves and very branched, Mikania had 6 leaves to vining, the goldenrod was branching and the Boston fern was growing vigorously. The degree of weed control was visually rated 20 to 24 days after treatment (DAT). Control ratings are based on the scale of 0 to 100 where 0 = no effect, 20 = minimal control and 100 = complete control. Variations in the results of the tests could be due to the fact that the tests were run at different times of the year or due to the stage of growth of the weeds. Test results are given in Tables 4 to 8.

Table 4

| | | Control Rating 24 DAT | |
|---|---|---|---|
| Treatment | Rate g/ha | Asystasia intrusa* | Nephrolepis exalata |
| Compound I | 64 | 60 | 80 |
| | 32 | 50 | 60 |
| | 16 | 40 | 40 |
| | 8 | 30 | 0 |
| | 4 | 20 | 0 |
| Check | 0 | 0 | 0 |

*At 4-leaf stage

Table 5

| | | Control Rating 20 DAT | |
|---|---|---|---|
| Treatment | Rate g/ha | Mikania[1] cordata | Asystasia[2] intrusa |
| Compound I | 64 | 30 | 100 |
| | 32 | 0 | 90 |
| | 16 | 0 | 60 |
| | 8 | 0 | 0 |
| Check | 0 | 0 | 0 |

[1] At 6 to 8-leaf stage
[2] At 6 to 9-leaf stage

Table 6

| | | Control Rating 20 DAT | | |
|---|---|---|---|---|
| Treatment | Rate g/ha | Mikania* cordata | Asystasia* intrusa | Goldenrod* |
| Compound I | 64 | 0 | 30 | - |
| | 32 | 0 | 0 | 30 |
| | 16 | 0 | 0 | 30 |
| | 8 | 0 | 0 | - |
| Check | 0 | 0 | 0 | 0 |

- = not determined

*Profusely branching

Table 7

| | | Control Rating 22 DAT |
|---|---|---|
| Treatment | Rate g/ha | Mikania* cordata |
| Compound I | 64 | 20 |
| | 32 | 0 |
| | 16 | 0 |
| | 8 | 0 |
| Check | 0 | 0 |

*Vining and profusely branching.

Table 8

| Treatment | Rate g/ha | Control Rating 22 DAT Mikania* cordata | Lalang |
|---|---|---|---|
| Compound I | 64 | 20 | 40 |
| | 32 | 0 | 20 |
| | 16 | 0 | 20 |
| Check | 0 | 0 | 0 |

Test 5

Sugarcane (Saccharum spp.) cuttings which had been pre-sprouted were planted in 15-cm plastic pots. Narrowleaf panicum (Panicum maximum), large crabgrass (Digitaria sanguinalis), guineagrass (P. maximum) and itchgrass (Rottboellia exaltata) were seeded in individual 15-cm plastic pots.

The pots were treated postemergence when the plants had 3 to 4 leaves with 2 to 3 tillers with Compound I formulated in a non-phytotoxic carrier made up of acetone-humectant-water-surfactant (22:1:1:0.05 v/v). The degree of crop injury and weed control was visually rated 27 days after treatment (DAT). Plant injury ratings are based on the scale of 0 to 100 where 0 = no effect, 20 = minimal injury and 100 = complete control. Test results are shown in Table 9.

Table 9

| | | Plant Injury Rating 27 DAT | | | | |
|---|---|---|---|---|---|---|
| Treatment | Rate g/ha | Sugarcane | Narrowleaf panicum | Large Crabgrass | Guinea Grass | Itchgrass |
| Compound I | 64 | 30 | 100 | 100 | 100 | 100 |
| | 32 | 20 | 100 | 80 | 80 | 100 |
| | 16 | 20 | 100 | 60 | 70 | 80 |
| | 8 | 10 | 70 | 30 | 30 | 80 |
| Check | 0 | 0 | 0 | 0 | 0 | 0 |

Test 6

The object of this test was to evaluate the effect of surfactants and plant growth stage on the efficacy of the compound of this invention. Plastic windowsill pots were filled with planting medium and seeded with yellow nutsedge (Cyperus esculentus), large crabgrass (Digitaria sanguinalis), goosegrass (Elsusine indica), bermudagrass (Cynodon dactylon), broadleaf signalgrass (Brachiara platyphalla), smooth crabgrass (Digitaria ischaemum), guinea grass (Panicum maximum) and johnsongrass (Sorghum halapense). Coffee (Coffea spp) plants with 2 to 4 branches were included in one test.

Plants were treated postemergence with Compound I formulated with different surfactants or a non-phytotoxic carrier made up of acetone-humectant-water-surfactant. The results showed that addition of a surfactant could improve the weed control, while maintaining safety to coffee.

Test 7

Individual 11.4-cm plastic pots filled with planting medium were seeded with rape, wild radish (Raphanus raphanistruna). tansy mustard (Descurainia pinnata), ball mustard (Neslia paniculata), black

mustard (Brassica nigra), yellow mustard, brown mustard, lentils, safflower, flax, sunflower, peanut, vetch, alfalfa, field bean, pea, violet (Viola spp), Kochia (Kochia spp), Russian thistle (Salsola spp), Matricaria spp, groundsel (Senecio spp), shepherdspurse (Capsella bursa-pastoris), nightshade (Solanum spp), sowthistle (Sonchus spp), sugar beet, wheat, "Bonanza" barley and "Morex" barley, Kentucky bluegrass (Poa pratensis), red clover (Trifolium pratense), dandelion (Taraxacum spp) and buckhorn plantain (Plantago lanceolata).

Plants were treated postemergence with the compound formulated with a non-phytotoxic carrier. Plants were visually rated 18 days after treatment (DAT). Response ratings were based on the scale of 0 to 100 where 0 = no effect, 20 = minimal injury and 100 = complete control. Test results are shown in Table 10.

Table 10

| | Plant Injury Rating 18 DAT | | |
|---|---|---|---|
| | Compound I | | Check |
| Rate (g/ha) | 32 | 16 | 0 |
| Rape | 100 | 80 | 0 |
| Wild radish | 100 | 100 | 0 |
| Wild mustard | 100 | 100 | 0 |
| Tansy mustard | 100 | 100 | 0 |
| Ball mustard | 100 | 100 | 0 |
| Black mustard | 100 | 100 | 0 |
| Yellow mustard | 100 | 100 | 0 |
| Brown mustard | 100 | 100 | 0 |
| Onicutal mustard | 100 | 100 | 0 |
| Lentils | 100 | 100 | 0 |
| Safflower | 100 | 100 | 0 |
| Flax | 100 | 100 | 0 |
| Sunflower | 100 | 100 | 0 |
| Peanut | 100 | 100 | 0 |
| Vetch | 100 | 100 | 0 |
| Alfalfa | 100 | 100 | 0 |
| Field bean | 100 | 100 | 0 |
| Pea | 100 | 100 | 0 |
| Viola | 100 | 100 | 0 |
| Kochia | 100 | 100 | 0 |
| Russian thistle | 100 | 100 | 0 |
| Matricaria | 100 | 100 | 0 |
| Groundsel | 100 | 100 | 0 |
| Sheperdspurse | 100 | 100 | 0 |
| Nightshade | 60 | 50 | 0 |
| Sowthistle | 100 | 100 | 0 |
| Sugar beet | 100 | 100 | 0 |
| Wheat | 100 | 100 | 0 |
| Bonanza barley | 100 | 90 | 0 |
| Morex barley | 100 | 90 | 0 |
| Kentucky bluegrass | 100 | 100 | 0 |
| Red clover | 100 | 100 | 0 |
| Dandelion | 100 | 100 | 0 |
| Buckhorn plantain | 50 | 30 | 0 |

## Test 8

The object of this test was to evaluate the compound of this invention for postemergence safety to pineapple, turfs and weed control. Climax timothy, Kentucky bluegrass, forage fescue, Kentucky fescue,

bermudagrass, bentgrass, annual bluegrass, bahiagrass, purple nutsedge, yellow nutsedge, large crabgrass, perennial ryegrass, annual ryegrass, galium and pineapple were sprayed postemergence with the compound in a non-phytotoxic solvent. Plants were visually rated 36 days after treatment (DAT). Injury ratings were based on the scale of 0 to 100 where 0 = no effect, 20 = minimal effect and 100 = complete control. Test results are shown in Table 11.

Table 11

| | Plant Injury Rating 36 DAT | | |
|---|---|---|---|
| | Compound I | | Check |
| Rate (g/ha) | 32 | 16 | 0 |
| Climax timothy | 100 | 100 | 0 |
| Kentucky bluegrass | 100 | 100 | 0 |
| Forage fescue | 100 | 100 | 0 |
| Galium | 100 | 100 | 0 |
| Pineapple | 0 | 0 | 0 |
| Kentucky fescue | 100 | 100 | 0 |
| Bermudagrass | 0 | 0 | 0 |
| Bentgrass | 100 | 70 | 0 |
| Annual bluegrass | 100 | 100 | 0 |
| Bahiagrass | 100 | 70 | 0 |
| Purple nutsedge | 100 | 50 | 0 |
| Yellow nutsedge | 100 | 60 | 0 |
| Large crabgrass | 80 | 60 | 0 |
| Perennial ryegrass | 100 | 100 | 0 |
| Annual ryegrass | 100 | 100 | 0 |

Test 9

Plastic windowsill pots containing planting medium were seeded with bahiagrass, bindweed (Convolvulus spp), guinea grass (Panicum maximum), large crabgrass (Digitaria sanguinalis) and pigweed (Amaranthus spp). Individual 15-cm plastic pots contained sandbur (Cenchrus echinatus), Mikania cordata, Boston fern (Nephrolepis exalata), quackgrass (Agropyron repens), elephantgrass (Pennisetum purpureum) and sowthistle (Sonchus spp) plants.

Plants were treated postemergence with Compound I formulated in a non-phytotoxic solvent. Plants were visually rated 22 days after treatment (DAT). Injury and weed control ratings were based on the scale of 0 to 100 where 0 = no effect, 20 = minimal control and 100 = complete control. Test results are shown in Table 12.

Table 12

| | Plant Injury Rating 22 DAT | | | |
|---|---|---|---|---|
| | Compound I | | | Check |
| Rate (g/ha) | 32 | 16 | 8 | 0 |
| Sandbur | 100 | 100 | 100 | 0 |
| Mikanis cordata | 30 | 0 | 0 | 0 |
| Boston fern | 80 | 50 | 40 | 0 |
| Sowthistle | 100 | 70 | 60 | 0 |
| Quackgrass | 100 | 90 | 70 | 0 |
| Lalang | 20 | 0 | 0 | 0 |
| Elephantgrass | 70 | 50 | 0 | 0 |
| Bahia grass | - | 40 | - | 0 |
| Bindweed | - | 70 | - | 0 |
| Guinea grass | - | 60 | - | 0 |
| Large crabgrass | - | 60 | - | 0 |
| Pigweed | - | 90 | - | 0 |
| Coffee | 0 | 0 | 0 | 0 |
| Sugarcane | 30 | 20 | 10 | 0 |
| - = not determined | | | | |

Test 10

The object of these tests was to evaluate the compound of this invention for postemergence safety to crops and weed control. Seedling and budded Macedine grapes were grown in 11.4-cm pots. Flax, green foxtail (Setaria viridis), "Morex" barley, wild oats (Avena fatua), "Era" wheat, annual ryegrass (Lolium multiflorum), wild radish (Raphanus raphanistrum), rape, sunflower, chickweed (Stellaria media) and wild buckwheat (Polygonum convolvulus) were seeded in windowsill pots.

The plants were sprayed postemergence with Compound I in a non-phytotoxic solvent. Plants were visually rated 15 and 27 days after treatment (DAT). Injury and weed control ratings were based on the scale of 0 to 100 where 0 = no effect, 20 minimal control and 100 = complete control. Test results are shown in Tables 13 and 14.

Table 13

| | | Plant Injury Rating 27 DAT | |
|---|---|---|---|
| | | Grapes | |
| Treatment | Rate g/ha | Seedling | Budded |
| Compound I | 32 | 20 | 10 |
| | 16 | 20 | 10 |
| | 8 | 10 | 0 |
| | 4 | 0 | 0 |
| | 2 | 0 | 0 |
| | 1 | 0 | 0 |
| Check | 0 | 0 | 0 |

Table 14

| | Plant Injury Rating 15 DAT | |
|---|---|---|
| | Compound I | Check |
| Rate (g/ha) | 16 | 0 |
| Flax | 100 | 0 |
| Green foxtail | 100 | 0 |
| Morex barley | 100 | 0 |
| Wild oats | 100 | 0 |
| Era wheat | 100 | 0 |
| Annual ryegrass | 100 | 0 |
| Wild radish | 100 | 0 |
| Rape | 100 | 0 |
| Sunflower | 100 | 0 |
| Chickweed | 100 | 0 |
| Wild buckwheat | 100 | 0 |

Test 11

Budded citrus seedlings were planted in 30-liter plastic pots which were also seeded with sandbur, guineagrass, narrowleaf panicum, common ragweed, yellow nutsedge, purple nutsedge and bindweed. Compound I was applied preemergence and postemergence to the weeds. Treatments were sprayed to simulate the trunk-to-trunk herbicide application method used in some citrus groves. The compound was also applied postemergence to weeds grown in windowsill flats. The flats were filled with planting medium and seeded with morningglory (Ipomoea spp.). purple nutsedge (Cyperus rotundus), pigweed (Amaranthus spp.), bindweed (Convolvulus spp.), narrowleaf panicum (Panicum maximum), common ragweed (Ambrosia artemisiifolia), sandbur (Cenchrus echinatus), itchgrass (Rottboellia exaltata), johnsongrass (Sorghum halepense), guineagrass (Panicum maximum), smooth crabgrass (Digitaria ischaemum), broadleaf signalgrass (Brachiaria platyphylla), bermudagrass (Cynodon dactylon), goosegrass (Eleusine indica), large crabgrass (Digitaria sanguinalis) and yellow nutsedge (Cyperus esculentus).

The compound was formulated and sprayed with 0.25% X-77 surfactant in water. Plants were visually rated 21 and 29 days after treatment and compared with appropriate controls. Injury ratings were based on the scale of 0 to 100, where 0 indicates no effect, 20 indicates minimum effect and 100 indicates complete control. Variations in the results of this test with others could be due to the fact that the tests were conducted at different times of the year. Test results are shown in Tables 15 and 16.

Table 15

| | Compound I | |
|---|---|---|
| Species | Preemergence 32 g/ha | Postemergence 32 g/ha |
| Citrus | 0 | 0 |
| Sandbur | 100 | 100 |
| Guineagrass | 100 | 100 |
| Narrowleaf panicum | 80 | 90 |
| Common ragweed | 20 | 0 |
| Yellow nutsedge | 100 | 100 |
| Purple nutsedge | 100 | 90 |
| Bindweed | 90 | 100 |

Table 16

| | Compound I |
|---|---|
| Species | Postemergence 32 g/ha |
| Morningglory | 90 |
| Purple nutsedge | 100 |
| Pigweed | 100 |
| Bindweed | 100 |
| Narrowleaf panicum | 90 |
| Common ragweed | 0 |
| Sandbur | 100 |
| Itchgrass | 100 |
| Johnsongrass | 100 |
| Guineagrass | 100 |
| Smooth crabgrass | 100 |
| Broadleaf signalgrass | 100 |
| Bermudagrass | 20 |
| Goosegrass | 90 |
| Large crabgrass | 80 |
| Yellow nutsedge | 80 |

Test 12

The object of this test was to evaluate the safety of sour lemon to the over-the-top application of Compound I and control of lantana (Lantana camara) and trumpetcreeper (Campsis radicans). Plants were sprayed postemergence with Compound I formulated in a non-phytotoxic solvent. Treated plants were visually rated 26 days after treatment and compared with appropriate controls. Injury ratings were based on the scale of 0 to 100, where 0 indicates no effect, 20 indicates minimal effect and 100 indicates complete control. Results are shown in Table 17.

Table 17

| | Compound I | | |
|---|---|---|---|
| | Postemergence | | |
| Species | 32 | 16 | 8 g/ha |
| Sour Lemon | 70 | 50 | 20 |
| Lantana | 30 | 10 | 0 |
| Trumpetcreeper | 40 | 20 | 0 |

Test 13

Conifer species (Loblolly pine) seedlings were transplanted into 15-cm plastic pots filled with planting medium. Compound I was applied postemergence to the loblolly pine seedlings. The compound was formulated in a non-phytotoxic spray solvent. Plans were visually rated 240 days after treatment (DAT) and compared with appropriate controls. Injury ratings are based on the scale of 0 to 100, where 0 indicates no effect, 20 indicates minimal injury and 100 indicates complete kill. Results are shown in Table 18.

Table 18

| | Plant Injury Rating 240 DAT | |
|---|---|---|
| Treatment | Rate (g/ha) | Loblolly pine |
| Compound I | 125 | 0 |
| | 64 | 0 |
| | 32 | 0 |
| | 16 | 0 |
| | 8 | 0 |
| Check | 0 | 0 |

**Claims**

1. A method for controlling undesired weeds in noncrop areas, fallow land, turfs, pastures, certain conifers and plantation crops which comprises applying to the locus a herbicidally effective amount of 2-[[-(4,6-dimethoxypyrimidin-2-yl)aminocarbonyl]-aminosulfonyl]-3-pyridinecarboxylic acid, methyl ester.

2. A method as in Claim 1, where the locus to be protected is a noncrop area.

3. A method as in Claim 1, where the locus to be protected is fallow land.

4. A method as in Claim 3, wherein after the application, a crop is planted during the next growing season.

5. A method as in Claim 4, wherein the crop is wheat or barley.

6. A method as in Claim 1 wherein the locus to be protected is a locus where turf and pasture grasses are grown.

7. A method as in Claim 1 wherein the locus to be protected is where a plantation crop is grown.

8. A method as in Claim 7 wherein the plantation crop is coffee.

9. A method of Claim 7 wherein the plantation crop is tea.

10. A method as in Claim 7 wherein the plantation crop is cocoa.

11. A method as in Claim 7 wherein the plantation crop is rubber or oil palm.

12. A method as in Claim 7 wherein the plantation crop is sugarcane.

13. A method as in Claim 7 wherein the plantation crop is banana.

14. A method as in Claim 7 wherein the plantation crop is pineapple.

15. A method as in Claim 1 wherein the locus to be protected is a conifer planting.

16. A method as in Claim 1 wherein the locus to be protected is a grape planting.

17. A method as in Claim 1 wherein the locus to be protected is a citrus planting.

18. A method as in Claim 1 wherein the locus to be protected is a fruit tree planting.

19. A method as in Claim 1 wherein the locus to be protected is a nut tree planting.

20. A 3-pyridinecarboxylic acid, methyl ester, represented by the formula II:

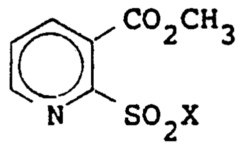

II

wherein

X is Cl, $NH_2$, $NHC(CH_3)_3$ and $HNC(O)OC_6H_5$.

21. The compound of Claim 20, 2-(aminosulfonyl)-3-pyridinecarboxylic acid, methyl ester.